# EUROPÄISCHE PATENTSCHRIFT

(11) **EP 0 625 918 B1**
(45) Veröffentlichungstag und Bekanntmachung des Hinweises auf die Patenterteilung: **02.09.1998**
(21) Anmeldenummer: 92924600.7
(22) Anmeldetag: 20.11.1992
(51) Int. Cl.: A61M 16/00, A61M 15/00

(54) **INHALATIONSEINRICHTUNG**
INHALATOR
INHALATEUR

(30) Priorität: 13.02.1992 DE 9201793 U; 07.07.1992 DE 9209050 U
(43) Veröffentlichungstag der Anmeldung: 30.11.1994
(73) Patentinhaber: von Schrader, Barthold, D-23570 Travemünde (DE)
(72) Erfinder: von Schrader, Barthold, D-23570 Travemünde (DE)
(74) Vertreter: Einsel, Robert, Dipl.-Ing.
(86) Internationale Anmeldenummer: EP9202679
(87) Internationale Veröffentlichungsnummer: WO9315783

(56) Entgegenhaltungen:
- EP-A- 0 140 838
- DE-U- 9 113 361
- US-A- 2 586 502
- US-A- 2 705 007
- US-A- 3 913 843

## Beschreibung

Die Erfindung betrifft eine Inhalationseinrichtung mit einem das Inhalationsmittel enthaltenden Gefäß und mit einem Deckel, der zur Verdampfung und Verteilung des Inhalationsmittels beiträgt.

Solche Inhalationsgeräte werden im allgemeinen mit einer sehr heißen Flüssigkeit gefüllt, in der das jeweils gewünschte Inhalationsmittel gelöst ist. Durch Spezialdüsen für Mund oder Nasenbeatmung gelangt ein das Inhalationsmittel enthaltender Dampf in die Atemwege. Diese bekannten Einrichtungen erfordern wegen der heißen Flüssigleit besondere Sorgfalt.

Es ist auch bekannt, das Inhalationsmittel in die Haut, z.B. den Brustbereich einzureiben und durch die Körperwarme verdampfen zu lassen. Diese an sich vorteilhafte Methode reizt gelegentlich die Haut und wird deshalb oft nicht akzeptiert.

Bei einer Inhalationseinrichtung mit einem ein Inhalationsmittel enthaltenden Gefäß ist es auch bekannt (US-A- 2,586,502), durch eine zentrale Öffnung im Deckel zu inhalieren und dem Inneren des Gefäßes über mehrere, ringförmig um die zentrale Öffnung im Deckel angeordnete Öffnungen Außenluft zuzuführen, wobei die zentrale Öffnung tiefer in das Gefäß ragt als die ringförmig angeordneten Öffnungen (4). Dort ist die zentrale Öffnung düsenartig verengt, um mittels eines Venturi-Effektes der schnell durch die Düse strömenden Luft aus dem flüssigen Inhalationsmittel über eine gesonderte seitliche Leitung Partikel des Inhalationsmittels sprayartig hinzuzufügen. Diese bekannte Einrichtung setzt der Atemluft wegen der düsenartigen Zentralöffnung großen Widerstand entgegen. Auch ist die bekannte Einrichtung nur für flüssiges Inhalationsmittel geeignet.

Der Erfindung liegt die Aufgabe zugrunde, das Inhalationsmittel ohne Verwendung heißer Flüssigkeiten und ohne Einreibung in die Haut den Atemwegen direkt in verträglicher Form zuzuführen und zugleich eine widerstandsarme Inhalation zu ermöglichen. Diese Aufgabe wird durch die im Anspruch 1 definierte Erfindung gelöst. Weiterbildungen der Erfindung sind in den abhängigen Ansprüchen gekennzeichnet.

Bei einem bevorzugten Ausführungsbeispiel der Erfindung ist der Deckel mit den beiden Öffnungssystemen an den Öffnungsbereich handelsüblicher, mit den jeweils gewünschten Inhalationsmitteln gefüllter, Behälter (beispielsweise unter dem Handelsnamen WICK VAPORUB erhältlich) angepaßt und wird auf diese dann geöffneten Behälter aufgeschraubt oder aufgesetzt. Im Betrieb einer solchen Inhalationseinrichtung aus Behälter und Deckel kann das Inhalationsmittel durch die strömende Atemluft oder einen willkürlichen, einstellbaren Luftstrom verdampft und in die Atemwege geleitet werden. Bei einem solchen Anwendungsfall wird die zentrale Öffnung über einen Atemschlauch mit Mundstück oder Nasenstück mit dem Patienten verbunden. Bei Einsaugung der Atemluft wird dann die Außenluft über die ringförmig um die Zentralöffnung angeordneten Öffnungen im Deckel angesaugt, verteilt sich über die Oberfläche des Inhalationsmittels im Behälter und gelangt mit den verdampften Teilen des Inhalationsmittels als Atemluft zum Patienten. Umgekehrt wird beim Ausatmen verdampftes Inhalationsmittel über die ringförmigen Öffnungen im Luftbereich um den Patienten verteilt und zugleich oder anschließend eingeatmet. Bei einer Kombination des Inhalationsgerätes gemäß der Erfindung mit einem handelsüblichen Atemventil, z.B. dem Atemventil AMBU der Fa. Heraeus, wird die zentrale Öffnung des Deckels mit dem Eingang des Atemventils verbunden und die ausgeatmete Luft direkt über das Ventil ausgeatmet.

Die Wirkung einer solchen Inhalationseinrichtung hat sich als günstig erwiesen, wenn der Öffnungsquerschnitt der zentralen Öffnung im wesentlichen gleich der Summe der Öffnungsquerschnitte der ringförmig angeordneten Öffnungen bemessen ist.

Die Einrichtung kann auch zur Anreicherung der Umgebungsluft genutzt werden, indem statt der Atemluft ein Gebläse verwendet wird wie beispielsweise ein Haartrockner oder ein diesem Anwendungszweck besonders angepaßtes Gebläse. Vorzugsweise wird ein solches Gebläse mit niedrigen Temperaturen von 50° betrieben, weil gemäß weiterer Erkenntnis Temperaturen um oder etwas über der Körpertemperatur für die Verdampfung des Inhalationsmittels günstig und ausreichend sind. Eine solche Einrichtung ist auch hinsichtlich des Energiebedarfs vorteilhaft.

Bei einer anderen Ausführungsform wird die günstige, oberhalb der Körpertemperatur liegende Verdampfungstemperatur über längere Zeit bewirkt oder aufrechterhalten, daß die benötigte Menge des Inhalationsmittels in einen doppelwandigen Behälter gegeben. Auch kann in den Behälter getrennt vom Inhalationsmittel ein Material eingefügt werden, das etwas oberhalb der Körpertemperatur seinen Zustand ändert, beispielsweise vom flüssigen in den festen Zustand wechselt und dabei Wärme soviel abgibt, wie es zur Aufrechterhaltung der Behälterinnentemperatur erforderlich ist. Ein solches Material ist z.B. das unter der Bezeichnung Tetradocanol bekannte Material. Es kann in den doppelwandigen Boden oder in eine Patrone eingefüllt in den Behälter gegeben werden.

Zur näheren Erläuterung der Erfindung werden im folgenden mehrere Ausführungsbeispiele anhand der Zeichnungen beschrieben. Diese zeigen in
- Fig. 1: einen Schnitt durch den Deckel und das Gefäß einer Inhalationseinrichtung gemäß der Erfindung,
- Fig. 2: eine Draufsicht zur Fig. 1,
- Fig. 3a und Fig. 3b: eine verstellbare Verschlußscheibe für den Deckel nach Fig.1
- Fig. 4: einen Deckel nach Fig. 1 ohne die eingesetzte, verstellbare Verschlußscheibe nach Fig. 3a oder Fig. 3b.
- Fig. 5a und Fig. 5b: eine Einrichtung mit Atemmaske und Atemschlauch,
- Fig. 6: eine Einrichtung mit Gebläse.
- Fig. 7a, Fig. 7b und Fig. 7c: eine Weiterbildung der Fig. 6 in verschiedenen Ansichten
- Fig. 8: eine Weiterbildung mit einem doppelwandigen Behälter.
- Fig. 9 a, b und c: einen Ventileinsatz für die Deckelanordnung.

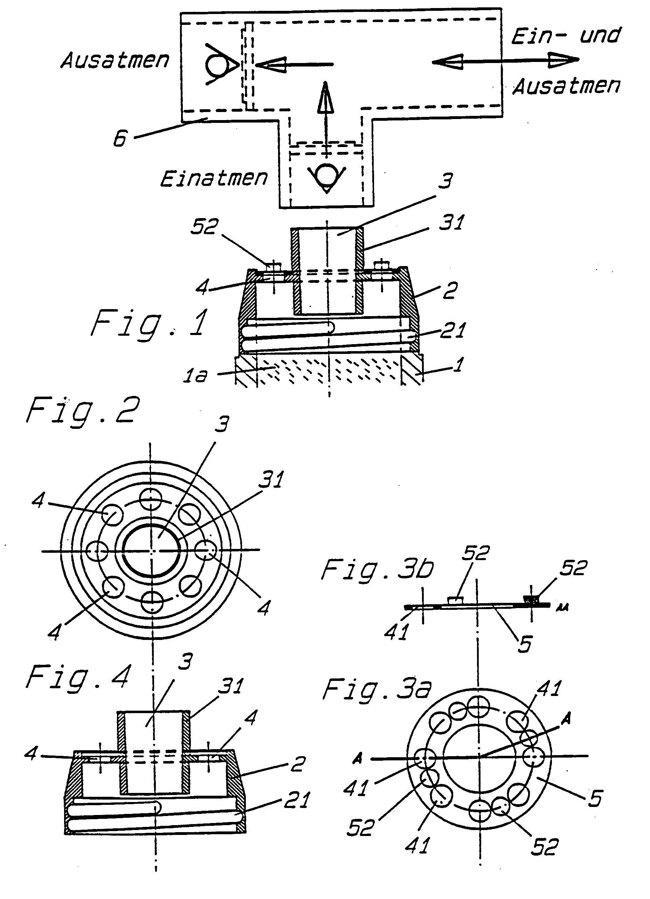

In Fig. 1 ist ein Gefäß 1 dargestellt, das mit einem vorzugsweise salbenartigen Inhalationsmittel 1a gefüllt ist. Solche Gefäße sind handelsüblich. Auf das Gefäß ist ein Deckel 2 aufgeschraubt. Dieser Deckel 2 kann bereits bei Lieferung des Gefäßes als Verschluß vorhanden sein. In diesem Fall ist das Gefäß 1 zunächst dicht verschlossen, beispielsweise durch eine nicht dargestellte Heißklebefolie, die vor Inbetriebnahme entfernt wird. Der Deckel 2 kann aber auch als Ersatz des ursprünglich vorhandenen Verschlußdeckels geliefert oder eingesetzt werden. Der Deckel 2 ist mit einer im wesentlichen zentral angeordneten Öffnung 3 versehen, die von mehreren Öffnungen 4 ringförmig umgeben ist. Die zentrale Öffnung 3 wird durch eine die scheibenförmige Deckelfläche durchdringende Röhre 31 gebildet. Dadurch wird erreicht, daß die Öffnung 3 bis nahe zur Oberfläche des Inhalationsmittels geführt werden kann. Die Röhre 31 kann fest sein, kann aber auch zur Einstellung des Abstandes von der Oberfläche längsverschieblich verstellbar angeordnet sein. Der Deckel 2 kann, wie dargestellt, mittels eines Schraubgewindes 21 mit dem Gefäß 1 verbindbar sein, das mit einem ähnlichen Gewinde versehen ist. Es kann aber auch steckbar sein. In diesem Fall ist es vorteilhaft, eine Raststellung vorzusehen.

Im einfachsten Fall ist die Röhre 31 mit Mund- bzw. Nasenadaptern versehen oder verbindbar. Bei einer solchen Benutzungsform hält der Benutzer den Behälter 1 in der Hand und atmet das Inhalationsmittel direkt über einen der Adapter ein. Durch die auf den Behälter 1 übergehende Handwärme wird die Verdampfung des Inhalationsmittels gefördert. Diese Benutzungsform ist für Reisezwecke besonders vorteilhaft.

Die zentrale Öffnung 3 kann mit einem gesonderten Atemventil 6 oder einer Atemmaske verbunden oder verbindbar sein. Solche Atemventile 6 werden beispielsweise von der Fa. Heraeus unter dem Handelsnamen AMBU angeboten. Die Röhre 31 des Deckels 2 ist so ausgebildet, daß das Atemventil 6 auf die Röhre 31 aufsteck- oder aufschraubbar ist. Ein solches Atemventil erlaubt bei Kombination mit dem Behälter 1 über den Deckel 2 das leichte Einatmen des mittels der Öffnungen 4 verdampften Inhalationsmittels und ein unbehindertes, leichtes Ausatmen über die Ventilstrecke des Atemventils 6.

Fig. 2 zeigt eine Draufsicht auf den in Fig. 1 dargestellten Deckel 2. Die ringförmig um die zentrale Öffnung 3 herum angeordneten Öffnungen 4 sind gruppenweise angeordnet. Dargestellt ist eine Anordnung von vier Gruppen mit je zwei Löchern 4. Eine solche Anordnung erlaubt eine Verwirbelung der strömenden Luft über der Oberfläche des Inhalationsmittels oder sogar eine einstellbare Dosierung. Das geschieht entweder durch Bemessung der Größe der Löcher 4 oder mit Hilfe der in Fig. 1 dargestellten zusätzlichen Scheibe 5, die in die Außenseite des Deckels 2 einsetzbar ist. Die Lagerung der Scheibe 5 im Deckel 2 ist so bemessen, daß die Scheibe 5 im Deckel 2 drehbar gelagert ist und so die Öffnungen 4 verschließbar macht. Die Scheibe 5 ist mit Vorsprüngen 52 und mit Löchern 41 versehen, die den Löchern 4 im Deckel 2 entsprechen. Die Scheibe 5 kann in Stellungen drehbar sein, in denen die Öffnungen 41 über den Öffnungen 4 liegen und somit freien Durchgang gewähren. In anderen Stellungen der Scheibe 5 werden die Öffnungen 4 durch die Wandung der Scheibe 5 ganz oder teilweise verdeckt, wodurch die resultierende Öffnung verkleinert oder sogar verschlossen wird. Zwischenstellungen ergeben also die jeweils gewünschte Größe der resultierenden Durchlaßöffnung. Die von der Scheibe 5 verdeckbaren Öffnungen 4 können auch nach Zahl und Größe von den Öffnungen 41 in der Scheibe 5 abweichen. Beispielsweise kann an der Stelle der Vorsprünge 52 der Scheibe 5 eine Öffnung 4 vorgesehen sein. Der Deckel 2 kann auch selbst als Ventil ausgebildet sein und über eine ventilfreie Atemmaske auf den Nutzer einwirken. In diesem Fall wird beim Einatmen der im Gefäß 1 befindlichen, mit dem Inhalationsmittel angereicherten Luft der Weg zum Patienten freigegeben, während dieser Weg beim Ausatmen wieder versperrt wird. Stattdessen wird ein weiteres im Deckel angeordnetes Ventil zum freien Luftraum geöffnet.

In Fig. 3a ist eine Scheibe 5 dargestellt, die mit vier Gruppen von je zwei Öffnungen 41 versehen ist. Zwischen je zwei Öffnungen 41 ist ein Vorsprung 52 angeordnet, der als Handhabe für die Verdrehung der in den Deckel 2 eingesetzten Scheibe 5 dienen kann. Fig. 3b zeigt die Scheibe 5 in geschnittener Seitenansicht. Durch Wahl des Schnitts entlang der Linien AA ist ein Vorsprung geschnitten und ein weiterer Vorsprung 52 in ungeschnittener Seitenansicht sichtbar.

Der Deckel 2 kann auch für andere Gefäße ausgebildet sein. Es ist auch möglich, Adapter für die Verbindung eines solchen einheitlichen Deckels mit anderen Gefäßen vorzusehen. Bei Verwendung für ein Gefäß, das für die Auflösung von Inhalationsmitteln in heißen Wasser bemessen ist, sollte über einer größeren Verdampfungsfläche ein sich nach oben zum Deckel 2 kegelförmig verjüngender Adapter vorgesehen sein.

Fig. 4 zeigt einen Deckel 2 mit den Öffnungen 3 und 4, bei dem die Scheibe 5 noch nicht eingesetzt ist.

Fig. 5a zeigt eine Einrichtung mit Deckel 2 und Atemmaske 61, die mit dem Adapter 31 verbindbar ist.

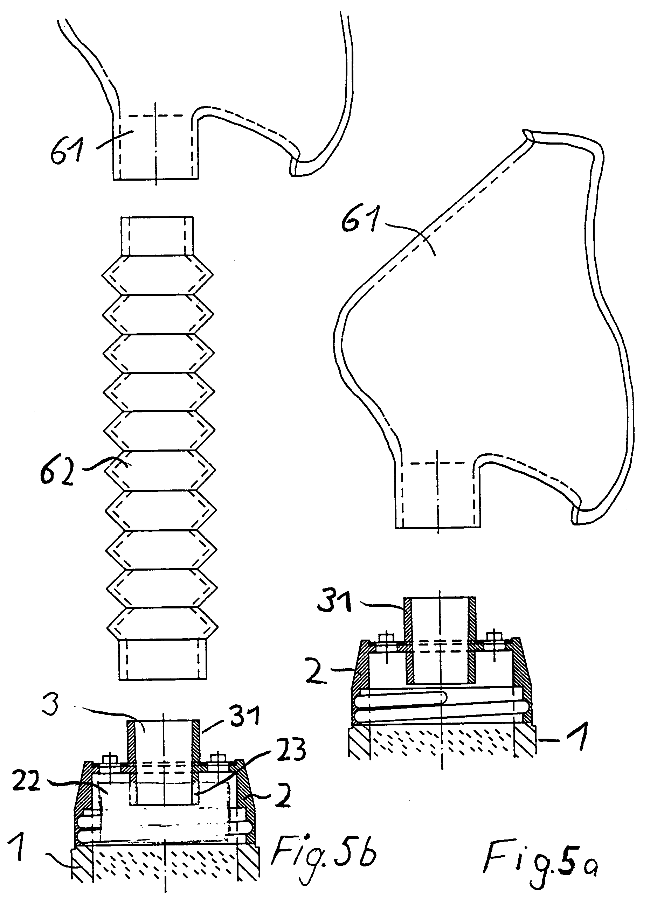

Fig. 5b zeigt eine Einrichtung mit Deckel 2, Atemmaske 61 und mit einem Schlauch 62, der für die Verbindung der Atemmaske 61 mit dem Adapter 3 ausgebildet ist.

Mit dem in den Figuren 1 - 5 dargestellten Deckel 2 wird ein optimaler Verdampfungseffekt dann erreicht, wenn die Oberfläche des salbenartigen Materials im Behälter 1 am größten ist. Zur Vergrößerung dieser Oberfläche kann -wie in Fig. 5b dargestellt-die für eine Inhalationsdauer benötigte Bedarfsmenge auf einen Träger 22 aufgetragen und dieser in den Deckel 2 oder den gefüllten oder leeren Behälter 1 eingesetzt werden.

Der Deckel 2 kann mit einer Aufnahme 23 für den Träger 22 versehen sein, so daß die Oberfläche dem Atemstrom besonders ausgesetzt ist. Der Träger 22 kann ein Formteil sein, kann aber auch aus formbarem Papier oder dergleichen geformt und in die Aufnahme 23 eingesetzt sein, z.B. in einen Schlitz im Rohr 31. Die jeweilige Bedarfsmenge kann einem Ersatzbehälter entnommen werden.

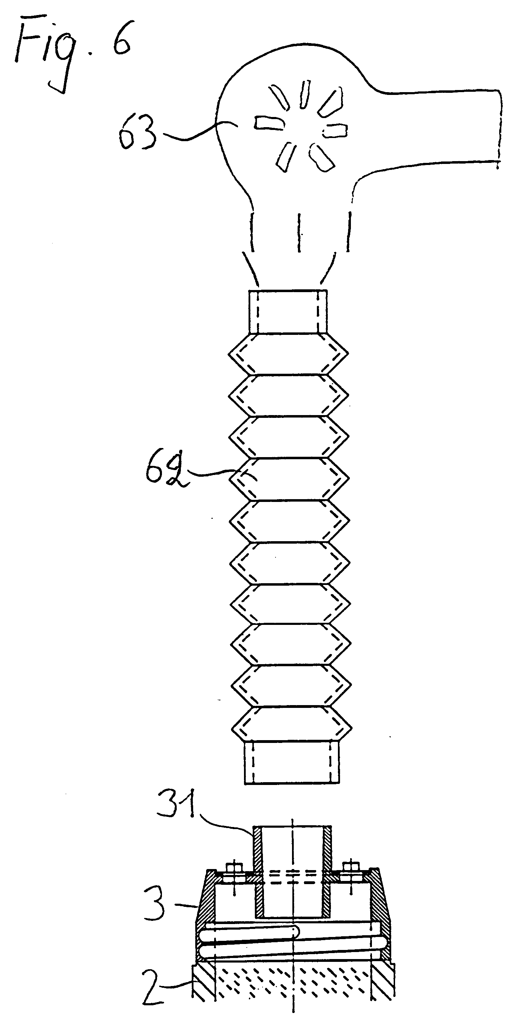

Fig. 6 zeigt die Verwendung der Einrichtung für die Anreicherung der Atemluft mit den Inhalationspartikeln. Zu diesem Zweck wird der Adapter 3 direkt oder über den Atemschlauch 62 mit einem Gebläse 63, z.B. einen Handelsüblichen Haartrockner oder dergl verbunden. Da solche Haartrockner speziell auch für Reisezwecke verfügbar sind und in vielen Hotels zur Zimmerausstattung gehören, kann mit der beschriebenen Einrichtung auch auf Reisen direkt oder indirekt inhaliert werden. Handelsübliche Haartrockner oder Gebläse können mit einer genormten Öffnung für den Anschluß der Einrichtung versehen werden. Falls eine solche genormte Öffnung nicht zur Verfügung steht, kann der Atemschlauch entsprechend variabel ausgebildet werden, um ihn an unterschiedliche Öffnung anschließbar zu machen. Dies kann entweder durch einen dehnbaren Anschluß oder durch ein Sortiment von Anschlüssen geschehen.

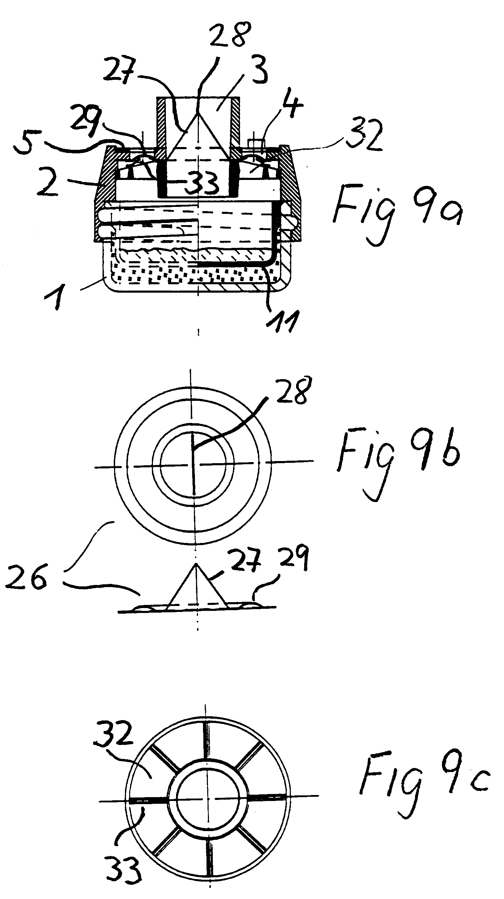

In Fig. 7a ist eine Inhalationseinrichtung mit Behälter 1 und Deckel 2 dargestellt, die mit einem Heizgebläse 63 zu einerkompakten Einheit zusammengefügt sind. Der Deckel 2 ist an seiner Außenwandung 24 mit einen Anschlag 25 versehen, der ringförmig ausgebildet ist, aber auch nur einzelne Vorsprünge aufweisen kann. Der Deckel 2 ist mit einem Gehäuse 64 des Gebläses 63 verbunden. Dieses Gehäuse 64 beinhaltet einerseits den Gebläsemotor 65 und andrerseits eine Luftführung 66 mit einer Heizeinrichtung 67 im Strömungsweg der Luft. Das Gehäuse 64 ist am Ende des Strömungsweges 66 an der dem Deckel 2 zugewandten Seite, in Fig. 7a an seiner Unterseite, mit einer an den Deckel 2 angepaßten Öffnung 68 versehen, die die Außenwandung 24 des Deckels 2 umfaßt. Die Länge der Röhre 31 des Deckels 2 und die Abmessungen des Gehäuses 64 sind so bemessen, daß die Röhre 31 die Wandung des Gehäuses 64 durch eine Öffnung 69 auf der der Öffnung 68 gegenüberliegenden Seite des Gehäuses 64 durchdringt.

Bei eingeschaltetem Gebläse 63 strömt die vom Motor 65 angesaugte Luft durch die Heizeinrichtung 67 in die Luftführung 66 des Gehäuses 64 und durch die Öffnungen 4 des Deckels 2 in den Behälter 1, vermischt sich dort mit den verdampften Inhalationspartikeln und gelangt durch die Röhre 31 zur Atemmaske 61 oder in die freie Umgebung.

Der Behälter 1 ist verschließbar. Es ist auch möglich, den Strömungsweg für die Luft und/oder das Luft-Partikel-Gemisch zu drosseln. In beiden Fällen können Motor 65 und Heizeinrichtung 67 zu hohe Temperaturen bewirken. Um eine Beschädigung des Gebläses 63 durch Überhitzung des Gehäuses 64 und/oder des Motors 65 zu verhindern, können Motor 65 und Heizeinrichtung 67 bei Luftstau oder überhitzung automatisch abgeschaltet oder heruntergeregelt werden.

In Fig. 7 ist eine Einrichtung gezeigt, bei der statt einer solchen automatischen Abschaltung das Gehäuse 64 selbst so ausgebildet ist, daß in jedem Fall eine Mindestluftströmung für das Gebläse 63 aufrechterhalten und damit eine Überhitzung vermieden wird. Zu diesem Zweck ist die Öffnung 69 im Gehäuse 64 etwas größer bemessen als die Außenwandung der das Gehäuse 64 durchdringen Röhre 31, so daß die vom Gebläse 63 erzeugte Luftströmung mit einem Mindestanteil durch eine zusätzliche Öffnung 691 parallel zur Röhre 31 aus dem Gehäuse 64 entweichen kann. Diese zusätzliche Öffnung 691 kann bei zusammengesetzter Einheit 2, 64 so bemessen oder geformt sein, daß die aus der Öffnung 691 austretende Luftströmung sich mit dem aus der Röhre 31 austretenden Inhalations-Luft-Partikel-Gemisch durchmischt. Da die so austretende Luft wärmer ist als das aus der Röhre 31 austretende Luft-Partikel-Gemisch, kann die Atemluft gewollt angewärmt werden. Die zusätzliche Öffnung 691 kann auch durch mehrere für die Durchmischung von Luft und Partikeln schräg zur Röhre 31 angeordnete Düsen gebildet oder ergänzt werden.

In Fig. 7b ist ein Schnitt durch den in Fig. 7a dargestellten Deckel 2 gezeigt. Die Röhre 31 ist auf ihrer in den Behälter 1 ragenden Seite mit einer schräg geschnittenen Öffnung 32 versehen. Durch diese Ausbildung wird die Verdampfung im Behälter gefördert. Fig. 7c zeigt eine Seitenansicht zur Fig. 7b mit den sichtbaren Schnittkanten der Röhre 31. Die Verdampfung kann noch mehr gefördert werden, wenn vor Gebrauch in den Behälter 1 poröse Steine aus Ton oder dergleichen gelegt werden. Diese können auch vorgewärmt sein.

Deckel 2 und Gehäuse 64 sind in Fig. 7a zwei getrennte, zusammenfügbare Teile. Der Deckel 2 kann aber auch mit dem Gehäuse 64 aus einem Stück gefertigt sein und so mit diesem eine konstruktive Einheit bilden, in die der Behälter 1 eingeschraubt wird. Motor 65 und Heizeinrichtung 67 können mit Netzspannung betrieben werden. Wegen der relativ niedrigen Temperaturen und der dadurch geringen Heizleistung ist es auch möglich, solchen Inhalationseinrichtungen aus Batterien, z.B. im Auto, zu betreiben. Hierfür eignet sich beispielsweise der in Automobilen vorgesehene Anschluß für Zigarettenanzünder.

Mit der in Fig. 7a dargestellten Einrichtung kann die Atemluft bettlägeriger Patienten in Räumen oder Raumbereichen mit den Aromastoffen der hierfür zusammengestellten oder geeigneten, salbenförmigen oder flüssigen Medikamente angereichert werden.

Fig. 8 zeigt einen Behälter 1 mit Deckel 2 gemäß Fig. 1, bei dem durch einen Einsatz 11 ein Gesamtbehälter 1, 11 mit doppelter Wandung erzielt ist. Einerseits wird allein durch diese doppelte Wandung 1, 11 die Temperatur des Inhalationsmittels 1a über längere Zeit im optimalen Verdampfungsbereich gehalten. Andrerseits ermöglicht diese doppelte Wandung die Nutzung eines die Wärme stabilisierenden Mittels 1b getrennt vom Inhalationsmittel 1a. Als Wärmestabilisierendes Mittel 1b ist ein Material vorgesehen, das Wärme aufnehmen und speichern kann und das im Stande ist, diese Wärme über einen längeren Zeitraum wieder abzugeben. Ein solches Material ist beispielsweise Klinkersteinmehl, das gesondert erhitzt und dann in den Behälter-Hohlraum 1c zwischen den Wandungen 1 und 11 eingefüllt wird. Vorzugsweise wird aber ein Material wie Tetradocanol verwendet, das bereits bei der Herstellung des Behälters 1, 11 in den Hohlraum 1c eingefüllt wird und dort verbleibt. Dieses Material hat den Vorteil, daß es knapp oberhalb der Körpertemperatur vom flüssigen Zustand in den festen Zustand übergeht und bei diesem Übergang im Bereich von etwa 38° über längere Zeit Wärme dieser Temperatur hält oder abgibt.

Wenn der Behälter 1, 11 mit dem im Hohlraum 1c befindlichen temperatursensiblen Material 1b zur Sterilisierung bei 100° gewaschen oder erhitzt wird, wird zugleich das temperatursensible Material 1b verflüssigt. Anschließend wird das Inhalationsmittel 1a eingefüllt und der Deckel 2 aufgeschraubt. Die Temperatur des Behälters 1, 11 mitsamt Inhalt 1a und 1b wird absinken, bis das temperatursensible Material 1b im Bereich von etwa 38° in den festen Zustand übergeht. In diesem Bereich wird dann die Temperatur von 38° für eine längere Zeit gehalten. Der Behälter 1 muß aber nicht immer auf 100° erwärmt werden, um den geschilderten Temperaturverlauf zu erreichen. Schon eine Erwärmung auf etwa 50° bewirkt eine Verflüssigung des Materials und die beschriebene Wärmehaltung und Wärmeabgabe bei erneutem Übergang in den festen Zustand.

Die wärmestabilsierende Wirkung des Tetradocanol ist auch bei einfach ausgebildeten Behältern 1 nach Fig 1 einsetzbar. In solchen Fällen wird dieses Material in Patronen oder dergleichen gefüllt, die dann in den mit Inhalationsmaterial 1a gefüllten Behälter 1 gelegt werden.

Bei Anwendungsfällen mit nur geringen Dosierungen kann es vorkommen, daß die stete Wärme zu große Verdunstungsverluste bewirkt, wenn keine Atemventileinrichtungen, z.B. gemäß Fig. 1, verwendet werden. Fig. 9 zeigt eine Deckelanordnung zur Vermeidung oder Herabsetzung von Verdunstungsverlusten. An der Innenseite des Deckels 2 ist eine kombinierte Membran- und Lippen-Dichtung 26, 27, 28, 29 vorgesehen. Diese kombinierte Dichtung verschließt den Behälter 1 solange, wie nicht über ihn eingeatmet wird. Beim Ausatmen und auch bei vorübergehendem Nichtgebrauch ist der Behälter 1 praktisch verschlossen. Zu diesem Zweck ist eine einteilige Membran 26 mit einem in die zentrale Öffnung 3 des Deckels 2 ragenden Kegel 27 vorgesehen, der mit einem die Lippendichtung bildenden Schlitz 28 versehen ist. Im Bereich der Öffnungen 4 des Deckels 3 ist die Membran 26 mit Buckeln 29 ausgeformt, die im Ruhezustand die Öffnungen 3 verschließen.

Auf der dem Deckel 2 abgewandten Seite der Membran 26 ist ein Stabilisierungsgitter 32 angeordnet und fest mit dem Deckel 2 verbunden, das die Membran 26 stützend hält und ggf zusätzlich mit Federn 33 die Buckel 29 gegen die Öffnungen 4 drückt, um diese sicher zu verschließen.

In Fig. 9b ist die Membran 26 in Draufsicht dargestellt, um den Schlitz 28 deutlich zu machen. Fig. 9c zeigt in Draufsicht das Stabilisierungsgitter 32.

Beim Einatmen der Luft durch die Öffnung 3 biegt sich die Membran 26 an ihrem Randbereich nach unten und läßt Luft über das angewärmte Inhalationsmaterial 1a strömen. Dabei wird diese Luft durch die Öffnungen 4, die Buckel 29 und den abgebogenen Randbereich der Membran verwirbelt und gelangt unter Mitnahme von Spuren des Inhalationsmaterials 1a durch den Schlitz 28 des Kegels 27 der Membran 26 durch die Öffnung 3 zur inhalierenden Person. Da die in den Behälter 1 eindringende Luft von der Außenseite der Membran 26 an den angewärmten Wandungen des Behälters 1 entlangstreicht, wird sie schon vor Erreichen des Inhalationsmaterials angewärmt. Die Verwirbelung der angewärmten Luft mit den Spuren oder Partikeln des Inhalationsmaterials 1a begünstigt die gute Wirkung der Inhalation.

Die Membraneinrichtung kann zusammen mit der Steuerscheibe 5 eingesetzt werden, kann aber auch ohne diese Scheibe 5 verwendet werden.

## Patentansprüche

1. Inhalationseinrichtung mit einem Inhalationsmittel enthaltenden Gefäß (1), mit einem zur Verdampfung des Inhalationsmittels beitragenden Deckel (2), mit einer im wesentlichen zentralen Öffnung (3) im Deckel (2) und mehreren, im wesentlichen ringförmig um die zentrale Öffnung (3) im Deckel (2) angeordneten Öffnungen (4), wobei die zentrale Öffnung (3) tiefer in das Gefäß (1) ragt als die ringförmig angeordneten Öffnungen (4), wobei der Öffnungsquerschnitt der zentralen Öffnung (3) im wesentlichen gleich der Summe der Öffnungsquerschnitte der ringförmig angeordneten Öffnungen (4) ist, und wobei die zentrale Öffnung (3), die ringförmig angeordneten Öffnungen (4) und der Innenraum des Behälters (1) einen Atemweg bilden.

2. Inhalationseinrichtung nach Anspruch 1, **dadurch gekennzeichnet, daß** der Deckel an seiner Außenseite durch eine den Inhalt des Behälters (1) verdunstungsdicht abschließende Scheibe (5) verschlossen oder verschließbar ist.

3. Inhalationseinrichtung nach Anspruch 2, **dadurch gekennzeichnet, daß** die Scheibe (5) relativ zum Deckel (2) verdrehbar ist und Löcher (41) aufweist, die mit den ringförmig angeordneten Öffnungen (4) im Deckel (2) einen Atemweg bilden.

4. Inhalationseinrichtung nach Anspruch 2 oder 3, **dadurch gekennzeichnet, daß** für die Aufnahme der Scheibe im Deckel (2) eine ringförmige Nut (8) angeordnet ist.

5. Inhalationseinrichtung nach einem der Ansprüche 1 - 4, **dadurch gekennzeichnet, daß** der Öffnung (3) eine Röhre (31) zugeordnet ist, die als Adapter für Mund- oder Nasenstücke oder für Atemmasken ausgebildet ist.

6. Inhalationseinrichtung nach Anspruch 5, **dadurch gekennzeichnet, daß** in der Röhre (31) ein die Röhre verschließendes Ventil vorgesehen ist, das für die Einatmung des Patienten einen geringen Widerstand darstellt.

7. Inhalationseinrichtung nach Anspruch 1, **dadurch gekennzeichnet, daß** ein in Deckel (2) oder Behälter (1) einsetzbarer Träger (22) vorgesehen ist, auf den das Inhalationsmaterial zur Vergrößerung der Oberfläche dieses Inhalationsmaterials auftragbar ist.

8. Inhalationseinrichtung nach Anspruch 7, **dadurch gekennzeichnet, daß** an der Innenseite des Deckels (2) eine Halterung (23) für die Aufnahme des mit dem Inhalationsmaterial bestrichenen oder bedeckten Trägers (22) vorgesehen ist.

9. Inhalationseinrichtung nach Anspruch 5 und 8, **dadurch gekennzeichnet, daß** die Röhre (31) mit der Halterung (23) für den Träger (22) versehen ist.

10. Inhalationseinrichtung nach einem der Ansprüche 7-9, **dadurch gekennzeichnet, daß** der Träger (22) aus Papier, Pappe oder Krepp-Pappe besteht.

11. Inhalationseinrichtung nach einem der Ansprüche 1 - 10, **dadurch gekennzeichnet, daß** der Behälter (1) einen Innenbehälter (11) enthält und daß zwischen Behälter (1) und Innenbehälter (11) ein Hohlraum (1c) angeordnet ist.

12. Inhalationseinrichtung nach Anspruch 11, **dadurch gekennzeichnet, daß** der Hohlraum (1c) wärmeisolierend ausgebildet ist.

13. Inhalationseinrichtung nach Anspruch 11 oder 12, **dadurch gekennzeichnet, daß** der Hohlraum (1c) mit einem wärmespeichernden Material gefüllt ist.

14. Inhalationseinrichtung nach einem der Ansprüche 1 oder 2, **dadurch gekennzeichnet, daß** zwischen den Öffnungen (3,4) und dem Behälter (1) eine die Öffnungen verschließende Membran (26) so angeordnet ist, daß der Strömungsweg sich nur bei Einatmung öffnet.

15. Inhalationseinrichtung nach Anspruch 14, **dadurch gekennzeichnet, daß** die Membran (26) einen in die Öffnung (3) ragenden Vorsprung (27) mit einem Schlitz (28) zur Bildung einer Lippenmembran aufweist.

16. Inhalationseinrichtung nach Anspruch 15, **dadurch gekennzeichnet, daß** der Membran (26) ein abstützendes Gitter (32) zugeordnet ist, das auf die Membran oder Teile der Membran eine Federwirkung in Richtung auf die Öffnungen ausübt.

17. Inhalationseinrichtung nach einem der Ansprüche 1 - 16, **dadurch gekennzeichnet, daß** das Gefäß (1) und/oder der Innenbehälter (11) erwärmbar sind und daß für die Verdampfung des Inhalationsmaterials eine Betriebs-Temperatur vorgesehen ist, die um die Körpertemperatur oder eine geringfügig darüber liegende Temperatur wie 50°C bemessen ist.

## Claims

1. Inhaler device with a vessel (1) containing the substance to be inhaled and with a lid (2) helping to vaporize the substance to be inhaled ,which lid is provided with a substantially central opening (3) and several openings (4) arranged substantially in a ring around the central opening, whereby the central opening (3) is arranged deeper in the vessel (1) than the openings arranged in a ring, whereby the cross-section of the central opening (3) is substantially equal to the sum of the of the cross-sections of the openings (4) arranged in a ring, and whereby the central opening (3) together with the said several openings (4) positioned in a ring and the interior of the vessel (1) form an air passage for breathing.

2. Inhaler device according to claim 1, characterized in that the lid is locked or lockable on ist outside by a disc (5) sealing the vessel (1) contents evaporation-tight.

3. Inhaler device according to claim 2, characterized in that the disc (5) is rotatable relative to the lid (2) and comprises holes (41) which form an air passage in the lid (2) with the openings (4) in the lid (2).

4. Inhaler device according to claim 2 or 3, characterized in that a ring-shaped groove (8) is provided for the mounting of the disc (5) in the lid (2).

5. Inhaler device according to any one of claims 1 - 4, characterized in that the opening (3) is formed by a tube (31) which is shaped as an adapter for mouth and nose pieces or for breathing masks .

6. Inhaler device according to claim 5, characterized in that there is provided in the tube (31) a valve sealing the tube, which offers a slight resistance to the inhalationg of the patient.

7. Inhaler device according to claim 1, characterized in that on the inside of the lid (2) or vessel (1) there is provided a carrier (22) which is to be provided with the substance to be inhaled, for increasing the surface of this substance.

8. Inhaler device according to claim 7, characterized in that on the inside of the lid there is provided a holder (23) for the mounting of the carrier (22) which is to be provided with the substance to be inhaled.

9. Inhaler device according to claims 5 and 8, characterized in that the tube (31) is provided with the holder (23) ffor the mounting of the carrier (22).

10. Inhaler device according to any one of claims 7 - 9, characterized in that the carrier (22) consists of paper, beard or crepe-board.

11. Inhaler device according to any one of claims 1 - 10, characterized in that the vessel (1) contains an internal vessel (11) and between vessel (1) and internal vessel (11) a cavity (10) is provided.

12. Inhaler device according to claim 11, characterized in that the cavity (10) is constructed thermally insulating.

13. Inhaler device according to claim 11 or 12, characterized in that the cavity (10) is filled with a heat-storing material.

14. Inhaler device according to claim 1 or 2, characterized in that between the openings (3, 4) and the vessel (1) a membrane (26) sealing the openings is so arranged that the flow path opens only on inhalation.

15. Inhaler device according to claim 14, characterized in that the membrane (26) comprises a projection (27) projecting into the opening (3) with a slot (28) for forming a lip membrane.

16. Inhaler device according to claim 15, characterized in that there is assigned to the membrane (26) a supporting grating (32), which exerts on the membrane or parts of the membrane a spring effect in the direction of the openings.

17. Inhaler device according to any one of the claims 1 - 16, characterized in that the vessel (1) and/or the internal vessel (11) are constructed to be warmed, whereby for vaporizing the substance to be inhaled there is provided an operating temperature lying around or slightly above body temperature of about 50°.

## Revendications

1. Inhalateur comprenant un récipient (1), contenant un produit pour inhalations, et un bouchon (2) contribuant à la vaporisation de ce produit et présentant une ouverture (3) essentiellement centrale et plusieurs orifices (4) agencés essentiellement en anneau autour de l'ouverture centrale (3), laquelle s'engage plus profondément dans le récipient (1) que les orifices (4) agencés en anneau, la section de passage de l'ouverture centrale (3) étant essentiellement égale à la somme des sections de passage des orifices (4) agencés en anneau, et l'ouverture centrale (3), les orifices (4 agencés en anneau et l'espace intérieur du récipient (1) formant une voie de respiration.

2. Inhalateur selon la revendication 1, caractérisé en ce que le bouchon est fermé ou peut être fermé, sur son côté extérieur, par un disque (5) constituant une fermeture étanche à l'évaporation pour le contenu du récipient (1).

3. Inhalateur selon la revendication 2, caractérisé en ce que le disque (5) peut être tourné par rapport au bouchon (2) et présente des trous (41) qui forment une voie de respiration avec les orifices (4) agencés en anneau dans le bouchon (2).

4. Inhalateur selon la revendication 2 ou 3, caractérisé en ce qu'une rainure annulaire (8) est ménagée dans le bouchon (2) pour la réception du disque.

5. Inhalateur selon une des revendications 1 - 4, caractérisé en ce qu'une tubulure (31) est coordonnée à l'ouverture (3), tubulure qui est réalisée comme un adaptateur pour des embouchures ou des pièces d'adaptation au nez, ou pour des masques respiratoires.

6. Inhalateur selon la revendication 5, caractérisé en ce qu'une soupape permettant de fermer la tubulure (31) est prévue à l'intérieur de celle-ci, soupape qui, en position ouverte, oppose une faible résistance à l'inspiration du patient.

7. Inhalateur selon la revendication 1, caractérisé en ce qu'il est pourvu d'un support (22) pouvant être mis en place dans le bouchon (2) ou le récipient (1) et sur lequel peut être appliqué le produit pour inhalations, en vue de l'agrandissement de la surface de ce produit.

8. Inhalateur selon la revendication 7, caractérisé en ce qu'un élément de maintien (23) est prévu sur le côté intérieur du bouchon (2) pour la réception du support (22), sur lequel est étalé ou qui est recouvert de produit pour inhalations.

9. Inhalateur selon les revendications 5 et 8, caractérisé en ce que la tubulure (31) est pourvue de l'élément de maintien (23) pour le support (22).

10. Inhalateur selon une des revendications 7 - 9, caractérisé en ce que le support (22) est en papier, carton ou papier-crêpe.

11. Inhalateur selon une des revendications 1 - 10, caractérisé en ce que le récipient (1) contient un récipient interne (11) et qu'une cavité (1c) est formée entre le récipient (1) et le récipient interne (11).

12. Inhalateur selon la revendication 11, caractérisé en ce que la cavité (1c) est réalisée comme une cavité isolée thermiquement.

13. Inhalateur selon la revendication 11 ou 12, caractérisé en ce que la cavité (1c) est remplie d'un matériau accumulateur de chaleur.

14. Inhalateur selon la revendication 1 ou 2, caractérisé en ce qu'une membrane (26), disposée entre les passages formés par l'ouverture (3) et les orifices (4) et le récipient (1), est agencée de manière qu'elle ferme ces passages normalement et ouvre seulement la voie d'écoulement lors d'une inspiration.

15. Inhalateur selon la revendication 14, caractérisé en ce que la membrane (26) possède une protubérance (27) faisant saillie dans l'ouverture (3) et présentant une fente (28) pour la formation d'un joint à lèvres.

16. Inhalateur selon la revendication 15, caractérisé en ce qu'une grille d'appui (32) est coordonnée à la membrane (26) et exerce sur la membrane ou des parties de celle-ci un effet élastique en direction des passages.

17. Inhalateur selon une des revendications 1 - 16, caractérisé en ce que le récipient (1) et/ou le récipient interne (11) peuvent être chauffés et que la température de fonctionnement prévue pour la vaporisation du produit pour inhalations est de l'ordre de la température corporelle ou d'une température qui la dépasse faiblement et qui est par exemple de 50° C.
